# EUROPÄISCHE PATENTANMELDUNG

(11) **EP 2 384 781 A1**
(43) Veröffentlichungstag der Anmeldung: **09.11.2011**
(21) Anmeldenummer: 11165062.8
(22) Anmeldetag: 06.05.2011
(51) Int. Cl.: A61M 5/32, A61M 5/42

(54) **Punktionsnadelabdeckung mit einem Mikronadelarray**

(30) Priorität: 06.05.2010 DE 102010019614
(71) Anmelder: Immunservice GmbH, 20354 Hamburg (DE)
(72) Erfinder: Huland, Edith, 22391 Hamburg (DE)
(74) Vertreter: Vossius & Partner

(57) **Zusammenfassung**

Die vorliegende Erfindung betrifft eine Nadelabdeckung (42) für Punktionsnadeln, um eine sehmerverminderte Punktion zu ermöglichen. Die Nadelabdeckung umfasst eine Schutzhülle (1), um zumindest die Nadelspitze der Punktionsnadel (10) zu umhüllen, wobei die Schutzhülle eine zur Nadelspitze gerichtete Innenseite und eine von der Nadelspitze weg gerichtete Außenseite aufweist. Die Schutzhülle weist an ihrer Außenseite ein Array von Mikronadeln (2) auf, wobei vor der Punktion die Punktionsstelle mit dem Mikronadelarray behandelt werden kann. Insbesondere wird mit der Behandlung mittels Mikronadelarray eine schmerzlindernde Akupunkturwirkung erzielt oder die Möglichkeit ein Medikamentenabgabe geschaffen, wodurch die anschließende Punktion mit der Punktionsnadel unter vermindertem Schmerzempfinden appliziert werden kann.

## Beschreibung

Die vorliegende Erfindung betrifft eine Nadelabdeckung für Punktionsnadeln, insbesondere um eine schmerzverminderte Punktion zu ermöglichen. Zudem betrifft die Erfindung auch Punktionseinrichtung bzw. -system, beispielsweise eine Spritze, mit einer erfindungsgemäßen Punktionsnadelabdeckung mit Mikronadelarray. Insbesondere wird das Mikronadelarray zur Akupunktur verwendet, wodurch eine schmerzreduzierte Punktion ermöglicht wird. Alternativ oder kombiniert mit dem Akupunktureffekt kann das Mikronadelarray zur lokalen Applikation von Medikamenten zur Schmerzreduktion im unmittelbaren Punktionsbereich verwendet werden.

Als Trypanophobie oder (umgangssprachlich) Spritzenangst wird die irrationale Angst vor Injektionen bezeichnet. Dabei handelt es sich um eine spezifische Phobie, die als relativ häufig und als allgemein medizinisch relevant gilt. So ist es nicht selten, dass es bei Personen mit einer Spritzenphobie und solchen mit schlechten Vor-Erfahrungen bei einer Punktion zu Ohnmachtsanfällen kommt. Es gibt bereits zielgerichtete Therapien wobei zwischen einer Trypanophobie (Angst vor der Injektion als solche), einer Belonophobie (Angst vor Nadeln), einer Aichmophobie (Angst vor spitzen Gegenständen) oder einer Vaccinophobie (Angst vor Impfungen) unterschieden werden kann.

Insulinpflichtige Diabetiker mit "Spritzenangst" benötigen beispielsweise ein psychologisches Gutachten, bevor eventuell eine "Impfpistole" zur Verabreichung des Insulins von den Krankenkassen zur Verfügung gestellt werden kann. Es ist jedoch nicht immer möglich den zu injizierenden Ampulleninhalt anderweitig zu verabreichen.

So ist es eine Aufgabe der Vorliegenden Erfindung eine Vorrichtung bereitzustellen, mit der Punktionen verträglicher vorgenommen werden können, insbesondere auch bei Personen mit Spritzenangst.

Erfindungsgemäß wird dies dadurch erreicht, dass zumindest die Nadelspitze, vorzugsweise die gesamte Nadel von einer Nadelabdeckung verdeckt wird, die während der Punktion nicht abgenommen werden muss. Somit ist die Nadel für die Person, die die Punktion erhalten soll, vorzugsweise nie direkt sichtbar.

Insbesondere bezieht sich im Folgenden der Begriff der Punktion (von lat. punctum "Stich") auf den medizinischen Begriff der Punktion und somit auf das gezielte Setzen einer Nadel oder eines anderen spitzen Instrumentes. Die dabei aufgenommene Gewebsflüssigkeit oder -probe heißt Punktat. Eine Punktion kann zur Einspritzung in den Körper (z.B. intravenöse Injektion) oder zur Entnahme aus dem Körper (z.B. Lumbalpunktion) dienen. Hierzu verwendet man Hohlnadeln (z.B. Kanüle, Trokar).

Die Vorstellung, dass auch eine verdeckte Nadel während der Punktion die gleichen Schmerzen verursacht wie eine unverdeckte Nadel bereitet einigen Personen mit "Spritzenphobie" immer noch Unbehagen.

Auch dieses Problem wird durch die Erfindung gelöst, nämlich dadurch, dass die Spritzenangst weiter reduziert wird, indem die Punktion zusätzlich unter vermindertem Schmerzempfinden stattfindet.

Zwar sind Mittel bzw. Maßnahmen zur Reduzierung von Schmerzen bei einer Punktion bekannt, allerdings sind diese bei weitem nicht voll ausgeschöpft. Ein einfacher Fall einer Schmerzlinderung ist der Gebrauch eines Lokalanästhetikums, das beispielsweise mit einem Tupfer lokal auf der Haut aufgebracht wird. Dies ist jedoch nicht immer möglich und häufig unpraktisch. Zudem ist eine "Spritzenphobie" manchmal auch mit einer generellen Abneigung gegen die klassische Schulmedizin verbunden, sodass der Gebrauch eines zusätzlichen lokalen Betäubungs-Medikamentes eine neue Angst bzw. Unbehagen generieren kann.

Alternativ oder zusätzlich zu den klassischen schulmedizinischen Verfahren zur Schmerzlinderung wird häufig Akupunktur eingesetzt. Unter Akupunktur wird eine therapeutische Strategie verstanden, die im weitesten Sinn die Reizung von definierten Punkten am menschlichen Körper in verschiedener Weise (z.B. insbesondere mit Nadeln, aber beispielsweise auch durch Wärme oder Druck) verwendet. Die therapeutische Strategie ist auf dem Hintergrund chinesischer naturphilosophischer Anschauungen entstanden, die davon ausgeht, dass in präformierten Bahnen des Körpers, sogenannten Meridianen, Energie fließt. Störungen dieses Energieflusses sind nach Meinung der Traditionellen Chinesischen Medizin (TCM) die häufigsten Ursachen von Krankheiten. Die Akupunktur soll solche Energieblockaden oder Energiestörungen gezielt beeinflussen oder beheben können.

Die Akupunktur als Methode ist keineswegs standardisiert. Kontrovers wird beispielsweise die Tiefe des Nadeleinstichs oder die Dauer der Nadelung beurteilt, sowie der Einsatz von Elektro- und Laserstimulation. Die WHO bemüht sich derzeit um eine standardisierte Nomenklatur zur chinesischen Akupunktur. 1989 wurden in der "Standard Acupuncture Nomenclature" insgesamt 361 Akupunkturpunkte veröffentlicht. Die Akupunkturpunkte sind sehr uneinheitlich beschrieben. Anwendungsindikationen und Zahl der genannten Punkte können erheblich differieren, mehr als 2000 sind beschrieben.

Neben der traditionellen individuellen Akupunktur der TCM gibt es die formulierte Rezepturakupunktur, die locus dolendi oder trigger point Akupunktur (auch "Dawos" Akupunktur = für da wo's weh tut), die japanische (eher oberflächlichere) Akupunktur ohne De Qi Gefühl, die Akupunktur, die den Headschen Zonen folgt und die Mikrosystemakupunktur (Ohr, Fuß Zunge, Hand), sowie einige weitere Arten. Im Gegensatz dazu steht die TCM, die für jeden Patienten je nach Anamnese und Befunderhebung unterschiedliche Punktkombinationen vorsieht.

Im Allgemeinen dauert eine typische Akupunktursitzung etwa 20 bis 30 Minuten, dabei werden etwa 10 bis 16 Nadeln gestochen. Der Patient ist ruhig und entspannt. Auch nach der Akupunktur sollte der Patient noch eine Zeit lang entspannt verharren. Eine komplette Therapie umfasst in der Regel 10 bis 15 solcher Sitzungen. Es hat sich jedoch auch gezeigt, dass durch sehr kurze Anwendungen von Nadelstichen - beispielsweise im Bereich weniger Sekunden - bereits eine lokale schmerzlindere Wirkung erzielt werden kann.

Bei sachgemäßer Handhabung der Akupunktur treten wenig Nebenwirkungen auf. Bei Verwendung unzureichend sterilisierter Nadeln an verschiedenen Patienten können unter Umständen Krankheitserreger wie etwa Hepatitis B-, -C- und auch das HI-Virus übertragen werden. Es kann zur Ausbildung eines Hämatoms an der Einstichstelle, zu einer Blutung, zu Taubheitsgefühl oder zu Nadelungsschmerzen (2-4% der Patienten) kommen. Selten sind gefährliche Organverletzungen, wie etwa ein Pneumothorax oder eine unbeabsichtigte Verletzung der Lunge oder von Gefäßen (Bergqvist D: Vascular injuries caused by acupuncture. In: European journal of vascular and endovascular surgery: the official journal of the European Society for Vascular Surgery. 36, Nr. 2, August 2008, S. 160-3. doi:10.1016/j.ejvs.2008.04.004. PMID 18538597) oder Embolien und Fremdkörperreaktionen durch abgebrochene Akupunkturnadelteile. Silikonisierte Akupunkturnadeln können durch Ablagerung kleinster Mengen Silikon in der Haut Granulome verursachen.

Die Akupunktur ist national und international als Therapieoption für viele Indikationen weit verbreitet, aber medizinisch fachlich umstritten. Ergebnisse einer Konsensuskonferenz des National Institute of Health (JAMA 1998; 280 (17) 1518-15249) diskutieren eine Evidenz der Akupunkturbehandlung für bestimmte Formen der Übelkeit, Schmerzen und Fibromyalgie. Die in der Literatur genannten Indikationen gehen weit darüber hinaus und erfassen neben Krankheitssymptomen auch Suchtverhalten (Rauchen), kosmetische Indikationen ("face lifting"), Schmerzlinderung und vieles mehr. Zahlreiche Übersichtsarbeiten und die Aktivitäten der vielfältigen Akupunkturgesellschaften geben Hinweise auf die Vielfalt dieser Indikationen (z.B. Indikationsliste WHO, z.B. "Zusammenfassender Bericht des Arbeitsausschusses Ärztliche Behandlung" des Bundesausscllusses der Ärzte und Krankenkassen über die Beratungen der Jahre 1999 und 2000 zur Bewertung der Akupunktur gemäß §135 Abs. 1 SGB V "Akupunktur" veröffentlicht 2001).

Rostfreier Edelstahl ist das bevorzugte Material für herkömmliche Akupunktumadeln. Die Akupunkturnadeln sind üblicherweise etwa 10-50 mm lang, haben einen Durchmesser im Bereich von 0,10 bis 0,25 mm und einen Griff aus Metall oder Kunststoff und sind meist Einmalartikel. 1996 hat die FDA die Entscheidung getroffen, Akupunkturnadeln als Medizinprodukt nicht mehr in der Gefahrenklasse III sondern in die Gefahrenklasse II einzuordnen. Damit dürfen Akupunkturnadeln in den USA in der täglichen Praxis und nicht mehr nur experimentell eingesetzt werden. Besonders kurze Einzelnadeln mit Längen von 0,6 mm-1,3 mm werden bevorzugt für Kinder und bevorzugt für die Ohrakupunktur eingesetzt und können mit einzelnen runden etwa 9 mm messenden Pflastern befestigt werden.

So wünschenswert das Verfahren der Akupunktur zur Schmerzlinderung ist, so widersinnig erscheint der Einsatz von Akupunktur bei Personen mit Spritzenphobie, aufgrund der direkt sichtbaren Akupunkturnadeln zu sein.

Es ist ein weiterer Vorteil der vorliegenden Erfindung, dass eine Vorrichtung bzw. ein Verfahren bereitgestellt wird, wodurch das Schmerzempfinden von Personen mit Spritzenphobie während einer Punktion vermindert werden kann.

Die Erfindung ist aber auch für alle anderen Patienten hilfreich, die nicht unter einer Phobie leiden, denn unangenehm und schmerzhaft ist die Punktion mit Standardnadeln auch beim "Alltagspatienten". Die Beispiele hierfür sind zahlreich: Die Injektion in die Mundschleimhaut zur Leitungsanästhesie beim Zahnarzt ist ein unerfreuliches Ereignis, ebenso wie die Gabe einer Betäubungsspritze bei Wundversorgungen und allgemein (fast) alle Injektionen in der Pädiatrie. Kinder schätzen Spritzen und Nadeln und spürbare Nadelstiche nicht. Kindliche und erwachsene Diabetiker sind an Alternativen zu schmerzhaften Injektionen sehr interessiert. Hier bietet sich die Anwendung der Mikronadelarray-Abdeckung in Verbindung mit gängigen Systemen, u.a. einem verbreitet verwendeten sogenannten "Pen" an.

Darüber hinaus kann ein Einsatz in der Veterinärmedizin potentiell sinnvoll und notwendig sein. Grundlage hierfür ist, dass Tieren kein Schmerz zugefügt werden darf. In der Neufassung des deutschen Tierschutzgesetzes vom 18. Mai 2006 ist nach §5 ein mit Schmerzen verbundener Eingriff ohne Betäubung nicht erlaubt, wobei die Betäubung nur von einem Tierarzt durchgeführt werden darf. (Literatur: Untersuchungen zur Wirksamkeit und Gewebeverträglichkeit von Lokalanästhetika bei der Kastration männlicher Saugferkel, Inaugural-Dissertation zur Erlangung der tiermedizinischen Doktorwürde der Tierärztlichen Fakultät der Ludwig-Maximilians-Universität München, Anke Zankl, München 2007). Hierdurch kann ein schmerzlinderndes Injektionsverfahren notwendig werden.

Die der vorliegenden Erfindung zugrunde liegenden Aufgaben werden mit den Merkmalen der unabhängigen Ansprüche gelöst. Zudem werden bevorzugte Ausführungsformen der Erfindung in den abhängigen Ansprüchen sowie in den unten diskutierten Aspekten ohne und mit Bezug auf die beigefügten Zeichnungen beschrieben.

Die vorliegende Erfindung betrifft eine Nadelabdeckung für Punktionsnadeln, wobei die Nadelabdeckung vor, während und nach der Punktion die Nadel so abdeckt, dass der Patient die Nadel (bei bestimmungsgemäßer Verwendung) nie zu sehen bekommt. Insbesondere betrifft die vorliegende Erfindung eine Nadelabdeckung die das Schmerzempfinden der zu punktierenden Person während der Punktion vermindern kann. Die Schmerzlinderung während der Punktion wird vorzugsweise durch einen Akupunktureffekt erreicht, wobei auf der Nadelabdeckung eine Anordnung von Mikronadeln (nachfolgend auch "Mikronadelarray" bezeichnet) bereitgestellt wird. Alternativ oder kombiniert mit dem Akupunktureffekt kann das Mikronadelarray zur lokalen Applikation von Medikamenten zur Schmerzredulction im unmittelbaren Punktionsbereich verwendet werden. Dies kann beispielsweise mit einem Mikronadelarray, das massive Mikronadeln aufweist, dadurch erreicht werden, dass das Medikament zur lokalen Schmerzreduktion auf der Oberfläche den massiven Mikronadeln aufgetragen ist oder aufgetragen wird, wobei das Medikament dann zusammen mit den massiven Mikronadeln in die obersten Hautschichten eingebracht werden kann.

Zudem kann das Medikament auch unter Verwendung von porösen Mikronadeln in die obersten Hautschichten eingebracht werden, indem das Medikament entweder schon bei Auslieferung der Nadelabdeckung mit dem Medikament behandelt ist oder indem nach der Auslieferung der Nadelabdeckung das Medikaments in die porösen Mikronadeln eingebracht wird. Letzteres kann beispielsweise einfach erreicht werden, indem das Medikament durch Kapillarkräfte der porösen Mikronadeln "aufgesaugt" wird. Schließlich kann das Mikronadelarray entweder ausschließlich massive Mikronadeln, ausschließlich poröse Mikronadeln oder sowohl massive als auch poröse Mikronadeln aufweisen.

Zudem betrifft die Erfindung auch eine Punktionseinrichtung bzw. ein Punktionssystem, beispielsweise eine Spritze, mit einer erfindungsgemäßen Punktionsnadelabdeckung.

Gemäß einem ersten Aspekt betrifft die Erfindung eine Nadelabdeckung für eine Punktionsnadel mit einer Schutzhülle zum Abdecken bzw. Umhüllen zumindest einer Nadelspitze der Punktionsnadel, wobei die Schutzhülle eine zur Nadel, insbesondere zur Nadelspitze gerichtete Innenseite und eine von der Nadel bzw. Nadelspitze weg gerichtete Außenseite aufweist, wobei die Schutzhülle an zumindest einem Teil ihrer Außenseite ein Array von Mikronadeln aufweist. Vorzugsweise ist das Mikronadelarray so auf der Außenseite der Umhüllung angeordnet, dass es der Nadelspitze gegenüberliegt.

Vorzugsweise ist die Abdeckung bzw. Schutzhülle aus einem ausreichend steifen oder festen Material hergestellt, so dass sie die Nadelspitze wirksam gegen unbeabsichtigtes Stechen verbirgt. Gleichzeitig ist das Material der Schutzhülle vorzugsweise zumindest bereichsweise ausreichend flexibel, so dass die Schutzhülle vor, während und nach der Punktion sich derart verformen bzw. verschieben kann, dass die Nadel vorzugsweise nie direkt sichtbar wird.

Alternativ dazu oder in Kombination damit kann die Schutzhülle auch eine Einrichtung aufweisen, die beim Ausüben einer in Axialrichtung der Nadel wirkenden Druckkraft in Richtung der Nadelspitze beim Erreichen einer Grenzkraft die Umhüllung von der Nadelspitze entfernt, so dass die Nadelspitze freigelegt wird. Vorzugsweise ist die Grenzkraft größer als die Kraft, die zur Akupunktur bzw. Medikamentenabgabe mit dem Mikronadelarray erforderlich ist.

Die minimal invasiven Mikronadeln mit Größenordnungen im Mikrometerbereich üben lokale Stimulierung aus, ohne jedoch Schmerzrezeptoren zu erreichen. Herkömmliche Akupunkturnadeln sind demgegenüber mehrere Millimeter bis Zentimeter lang, ihre Anwendung beim Patienten/Probanden kann Schmerzen, Unbehagen und Angst auslösen. Außerdem sind Infektionen und Blutungen mögliche Nebenwirkungen. Das im Rahmen der Erfindung verwendeten Array hat minimal invasive Mikronadeln , die typischerweise 100µm bis500 µm lang sind und durchdringen die Haut ohne nennenswerte Aktivierung von Schmerzrezeptoren und ohne die durch Nadeln typische oft beobachtete Angstauslösung bei betroffenen Patienten. Nachweislich führen Mikronadeln aber durchaus zu lokaler Reizung der Hautpunkte und zu einem Durchdringen der obersten Hautbarriere. Dies ermöglicht den Akupunktureffekt.

Die erfindungsgemäß eingesetzten Mikronadelarrays sind in der Lage gleichzeitig und/oder gleichartig mehrere Hautpunkte zu stimulieren, ein deutlicher Vorteil gegenüber klassischen Akupunkturnadelmethoden. Es ist bekannt, dass auch die um definierte Akupunkturpunkte herumliegenden Hautpunkte günstige Effekte durch Akupunktur erzeugen können. Eine gleichzeitige Stimulierung kann daher eine Wirkungsverstärkung induzieren. Bei Mikronadelarrays ist dies schmerz- und risikoftei möglich.

Für den reinen Akupunktureffekt ist es ausreichend, wenn die Mikronadeln massiv sind. In Kombination damit oder alternativ dazu kann im Rahmen der vorliegenden Erfindung das Mikronadelarray als Träger für Medikamente dienen. Zum Beispiel kann zumindest teilweise auf der Oberfläche der Mikronadeln ein Medikament, z.B. ein Lokalanästhetikum, aufgebracht sein, das beim Applizieren der Mikronadeln in die Haut ebenfalls zumindest teilweise in die oberste Hautschicht eingebracht wird. Dadurch kann eine schnelle Wirkung des Lokalanästhetikums erreicht werden, wodurch das Schmerzempfinden während der Punktion zusätzlich vermindert werden kann. Für den Fall, dass größere Mengen des Medikaments in die Haut eingebracht werden sollen, kann es zudem vorteilhaft sein, wenn zumindest einige der Mikronadeln, vorzugsweise alle Mikronadeln porös oder hohl sind. Außerdem kann für größere Medikamentenmengen ein Reservoir an oder in der Schutzhülle vorgesehen sein, das mit dem Mikronadelarray verbunden ist, damit die Mikronadeln das Medikament aus dem Reservoir beziehen und an der zu punktierenden Stelle abgeben können. Der Akupunktureffekt bleibt davon unberührt.

Bevorzugt können hierzu Lokalanästhetika mit schnell eintretender Wirkung (z.B. Procain, Lidocain, Mepivacain, Prilocain, Articain, Etidocain, Benzocain, 2-Chlorprocain) Verwendung finden. Einigen Präparaten ist Adrenalin, Noradrenalin oder Phenylephrin beigemischt. Diese sogenannten Vasokonstriktoren verengen die Blutgefäße im Wirkbereich und senken so die Durchblutung. Dadurch wird die Resorption der Lokalanästhetika verringert und die Wirkdauer verlängert.

Die Mikronadeln auf dem Array der Umhüllung können erfindungsgemäß auch weitere Medikamente oder Substanzen enthalten, sofern es sinnvoll oder wünschenswert ist, diese im Zusammenhang mit einer nachfolgenden Punktion durch eine Injektionsnadel oder Punktionsnadel zu verbinden. Diese können in Kombination mit Lokalanästhetika oder auch einzeln verwendet werden. Kombiniert können in die Mikronadelarrays zum Beispiel Substanzen, die die Verteilung der Lokalanästhetika beeinflussen, wie z.B. Vasokonstriktoren (Adrenalin, Noradrenalin, Phenylephrin), oder Substanzen, die den pH Wert günstig beeinflussen, um die Wirksamkeit der Lokalanästhetika zu sichern und den Wirkungseintritt zu beschleunigen, gegeben werden. In die Mikronadelarrays können beispielsweise auch Medikamente gegeben werden, die die Infektabwehr der Haut verbessern oder die Abwehrlage der Haut schützen helfen, um dann durch die Mikronadelarrays auf die Punktionsstelle der Haut direkt vor der Punktion aufgebracht zu werden, bevor die Durchdringung mit der Punktionsnadel stattfindet, um beispielsweise abwehrschwache Patienten vor Hautinfekten in Verbindung mit der Punktion zu schützen. Hier können sich Antibiotika oder andere infektabweisende Stoffe eignen. Erfindungsgemäß vorteilhaft ist in diesem Fall die enge räumliche und zeitliche Abfolge der Kombination von Mikronadelarray mit Punktionsnadel.

Die Mikronadeln- können beispielsweise aus siliziumhaltigem Material, Metall, biologisch abbaubaren Materialien, insbesondere Polymeren, und anderen biologischen Materialien, Keramik, Maltose, Glas, oder Kunststoffen hergestellt sein. Die kleinsten Exemplare der erfindungsgemäßen Mikronadel sind vorzugsweise ein Tausendstel Millimeter groß. Für die Herstellung können beispielsweise Formvorlagen benutzt werden. So können Mikronadeln in verschieden Größen, Breiten und Profilen angefertigt werden und auf die geforderten Bedürfnisse ausgerichtet werden.

Experimente haben gezeigt, dass sowohl massive Nadeln als auch hohle Nadeln stabil genug sind, um Hautproben zu durchstechen. Beispielsweise ist es bereits bekannt, kleine Hohlnadeln einzusetzen, um Arzneimitteln in kleinsten Mengen gezielt und schmerzfrei zu verabreichen, z.B. Insulin oder Impfstoffe. Wegen ihrer geringen Länge treffen die Mikronadeln nicht oder nur auf wenige Nervenzellen und verursachen somit selbst keine Schmerzen, sondern können sogar zu Schmerzlinderung dienen.

Die Länge der Mikronadeln ist vorzugsweise so bemessen, dass sie nicht in die Dermis der Haut mit den Schmerzrezeptoren und Gefäße eindringen, wenn die Mikronadeln gegen die Haut gedrückt werden. Mikronadelarrays können für dünne Hautareale gezielt deutlich kürzere Nadeln (z.B. 70 µm bis 80 µm) einsetzen, die lediglich in die Epidermis eindringen und die die Schmerzrezeptoren nicht erreichen, und auch alternativ für dickere Hautschichten durch Wahl einer entsprechenden Länge der Mikronadeln selbst über 800 µm hinaus eine möglichst optimal überwiegend epidermale Eindringtiefe erreichen.

Um die weite Bandbreite von Anwendungsgebieten der vorliegenden Erfindung beispielhaft zu umreißen, werden im folgenden einige beispielhafte Anwendungsgebiete kurz diskutiert, ohne jedoch darauf beschränkt zu sein. Beispielsweise kann die erfindungsgemäße Vorrichtung besonders vorteilhaft sein bei Punktionen an empfindlichen Körperstellen: Botox-Injektionen in die Augennuskulatur; Schwellkörperautoinjektionstherapie (SKAT) bei der sich der der Patient unmittelbar vor dem geplanten Geschlechtsverkehr ein erektionsauslösendes Medikament direkt in die Schwellkörper des Penis injiziert, etc.. Die Vorrichtung der vorliegenden Erfindung kam auch für üblicherweise schmerzhafte Punktionen verwendet werde, die aufgrund der Größe der Punktionsnadel sehr schmerzhaft sind, beispielsweise bei der Punktion von Pleuraergüssen.

Die erfindungsgemäße Nabelabdeckung ist vorzugsweise so konstruiert, dass handelsübliche Punktionsnadeln nachträglich umhüllt werden können. Somit muss ein Patient oder ein behandelnder Arzt nicht ein komplettes Set bestehend aus Spritze, Nadel und Nadelabdeckung vorrätig haben, sondern kann die Spritzen und Nadeln, die üblicherweise verwendet werden, entsprechend nachrüsten.

Vorzugsweise umhüllt die Schutzhülle die Punktionsnadel vollständig. Die Schutzhülle ist zudem vorzugsweise vollständig fluiddicht, so dass die Nadelspitze die Schutzhülle beim Applizieren der Nadel durchsticht. Dies ist beispielsweise der Fall, wenn die Schutzhülle zusammen mit der Nadelspitze gegen ein Objekt gedrückt wird. Eine fluiddichte Schutzhülle bietet den Vorteil, dass die Nadel bis zum Gebrauch steril innerhalb der Schutzhülle gelagert werden kann.

Gemäß einer weiteren bevorzugten Ausführungsform kann die Schutzhülle eine Öffnung aufweist, durch die die Nadel sich erstreckt, wenn die Nadelspitze gegen ein Objekt gedrückt wird.

Erfindungsgemäß ist es bevorzugt bzw. ausreichend, wenn die Mikronadeln im Wesentlichen nur auf einer Teilfläche auf der Außenseite der Schutzhülle angebracht sind, an der sich die Nadelspitze durch die Schutzhülle erstreckt, wenn die Nadelspitze gegen ein Objekt gedrückt wird.

Die vorliegende Erfindung betrifft zudem ein Punktionssystem mit: (i) einer Spritze mit einem vorzugsweise zylindrischen Spritzenkörper und einem darin befindlichen Hohlraum, wobei ein Kolben in dem Hohlraum des Spritzenkörpers beweglich angeordneten ist; (ii) einer Punktionsnadel, die an der Spritze angebracht bzw. anbringbar ist: und (iii) einer erfindungsgemäßen Nadelabdeckung, wobei die Nadelabdeckung an der Spritze und/oder Punktionsnadel angebracht bzw. anbringbar ist, so dass die Schutzhülle die Punktionsnadel zumindest teilweise umhüllt.

Schließlich betrifft die vorliegende Erfindung auch ein Verfahren zum Applizieren einer vorzugsweise schmerzverminderten Haut-Punktion mit den Schritten: Bereitstellen eines erfindungsgemäßen Punktionssystems; leichtes Andrücken der Schutzhülle gegen die Haut, so dass die Mikronadeln in die Haut eindringen; anschließend stärkeres Andrücken der Schutzhülle gegen die Haut, so dass sich die Nadelspitze durch die Schutzhülle erstreckt und in Haut eindringen kann.

Die erfindungsgemäß eingesetzten Mikronadelarrays werden vorzugsweise in Gruppen von Mikronadeln auf einer gemeinsamen Grundfläche, einem Träger (der Außenseite der Nadelabdeckung) bereitgestellt und stimulieren dadurch mehr als einen Akupunkturpunkt gleichzeitig, ohne jedoch Schmerzen oder Blutung auszulösen, da die Nadellänge der Mikronadeln so bemessen ist, dass die Mikronadeln in die Epidermis der Haut penetrieren, diese jedoch nicht durchdringen und nicht die Dermis und Subcutis erreichen. Damit ist gewährleistet, dass Nerven und Blutgefäße nicht erreicht werden. Die Akupunktur erfolgt im wesentlichen schmerzfrei und blutungsfrei und erzielt dennoch den Akupunktureffekt, der mit dem Eindringen in die Haut einhergeht. Die Länge der Nadeln liegt vorzugsweise zwischen 30 µm bis 800 µm. Die Dicke der Epidermis ist sehr variabel und kann zwischen 0,03 mm und 4 mm liegen. Die äußere Schicht der Epidermis besteht aus dem Stratum Corneum mit verhornten, abgestorbenen Komeozyten (12-200 Zellschichten). Vorzugsweise wird je nach Einsatzort die Nadellänge abgestimmt, um beispielsweise Bereiche mit sehr dünnem Stratum Comeum oder solche mit dickem Stratum Comeum optimal durchdringen zu können und erfindungsgemäß die vitale Epidermis zu erreichen.

Die Anzahl der Mikronadeln auf dem Array wird vorzugsweise mit dem beabsichtigten Akupunktureffekt abgestimmt. Durch den Einsatz der Mikronadelarrays ist es beispielsweise möglich, eine Vielzahl von sehr nahe beieinander liegenden Akupunkturpunkten gleichzeitig zu stimulieren und Unsicherheiten bei der Identifikation optimaler Punkte zu beheben.

Akupunkturpunkte werden standardmäßig lediglich als Einzelpunkt gestochen. Jedoch ist es bekannt, dass auch Punktbereiche neben den typischen Akupunkturpunkten Wirksamkeit zeigen. Aus dieser Sicht ist eine Stimulierung der eigentlichen Akupunkturpunkte und naheliegender Umgebungspunkte, wie es mit dem erfindungsgemäß eingesetzten Mikronadelarray geschieht, sinnvoll.

Die Erfindung umfasst neben den oben beschriebenen Aspekten ebenfalls einzelne Merkmale in den Figuren, auch wenn sie dort im Zusammenhang mit anderen Merkmalen gezeigt sind und/oder vorstehend oder nachfolgend nicht genannt sind. Die Erfindung umfasst ebenfalls Ausfiihrungsformen mit jeglicher Kombination von Merkmalen, die vorstehend oder nachfolgend zu verschiedenen Ausfühmngsformen genannt oder gezeigt sind.

Im Folgenden werden bevorzugte Ausführungsformen der vorliegenden Erfindung unter Bezugnahme auf die Figuren ausführlich beschrieben. Es zeigen:
- Fig. 1: eine Spritze mit einer erfindungsgemäßen Nadelabdeckung, bei der die Schutzhülle von der Nadel durchstochen werden kann; und
- Fig. 2: eine Spritze ähnlich der in Fig. 1 gezeigten, jedoch mit einer Öffnung durch die sich die Nadelspitze während der Applikation hindurch erstrecken kann.

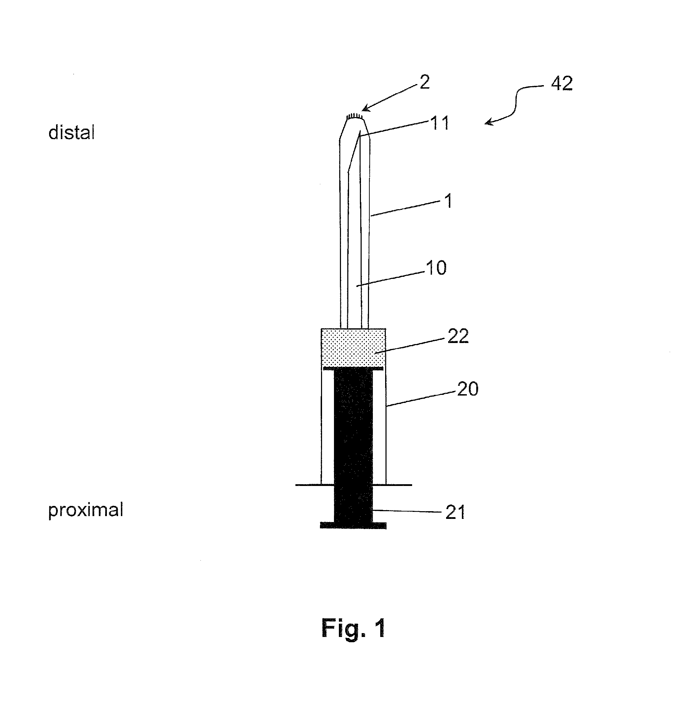

Fig. 1 zeigt eine erste Ausführungsform einer erfindungsgemäßen Nadelabdeckung 42 mit einer Schutzhülle 1, die eine auf einer Spritze angebrachte Punktionsnadel 10 (beispielsweise Injektionsnadel) vollständig umhüllt. Bei der dargestellten Spritze kann es sich um eine handelsübliche Kunststoffspritze oder Glasspritze handeln. Eine Spritze umfasst im Wesentlichen zwei Hauptbestandteile; einen Spritzenkörper 20 mit einem distalen und einem proximalen Ende und einem darin befindlichen Hohlraum, in dem ein Kolben 21 angeordnet ist, der in axialer Richtung des Spritzenkörpers 20 verschiebbar ist. Durch verschieben des Kolbens 21 kann ein Fluid 22 in den Hohlraum eingezogen bzw. aus dem Hohlraum herausgedrückt werden. Zudem ist üblicherweise am distalen Ende der Spritze eine Düse angebracht, vorzugsweise in Form eines Luer-Lock Konus ("männlicher" Teil der Luer-Lock Verbindung), um daran beispielsweise Schläuche bzw. Punktionsnadeln 10, usw. mit entsprechendem Luer-Lock Innenkegel ("weiblicher Teil" der Luer-Lock Verbindung) anzubringen. Im Folgenden wird das Ende der Punktionsnadel 10, das an der Spritze angebracht wird, als proximales Ende und das Ende mit der Nadelspitze 11 als distales Ende bezeichnet (siehe Fig. 1).

Unter dem Begriff "Luer-Lock" wird teilweise auch zwischen einer einfachen Version ohne Schraubgewinde und einer Version mit Schraubgewinde unterschieden. Bei der Version ohne Schraubgewinde wird die Spritzendüse häufig als Luer-Ansatz, Luer-Steck o.a. "Luer Slip" bezeichnet. Wenn der Konus zur Sicherung bzw. Verriegelung der Verbindung gegen versehentliches Lösen durch ein Gewinde mit Überwurfmutter erweitert ist, bezeichnet man das System häufig auch als Luer-Lock. Die Verbindung schließt und öffnet mit einer halben Drehung.

Die erfindungsgemäße Nadelabdeckung 42 umhüllt mit der Schutzhülle 1 vorzugsweise die komplette Punktionsnadel 10, so dass ein Patient die Nadeln 10 vor einer Punktion nicht zu sehen bekommt. Die erfindungsgemäße Nadelabdeckung 42 kann an ihrem proximalen Ende entweder direkt am Luer-Lock der Punktionsnadel 10 angebracht werden oder über einen entsprechenden Kupplungsmechanismus an der Spritze. Möglich sind auch Befestigungsmechanismen, die die erfindungsgemäße Nadelabdeckung 42 sowohl an dem Spritzenkörper 20 als auch an der Nadel befestigen.

Die Schutzhülle 1 der erfindungsgemäßen Nadelabdeckung 42 weist eine zur Nadel gerichtete Innenseite und eine gegenüberliegende, von der Nadel weg gerichtete Außenseite auf. Zudem weist die erfinduligsgemäße Nadelabdeckung 42 vorzugsweise zumindest am distalen Ende der Schutzhülle 1 ein Array mit Mikronadeln 2 auf. Hierbei ist zu beachten, dass die in Fig. 1 dargestellte Ausführungsform nicht maßstabsgetreu ist, und insbesondere die Mikronadeln 2 des Mikronadelarrays stark vergrößert dargestellt sind. Vorzugsweise enthält das Mikronadelarray eine Vielzahl von Mikronadeln 2, wobei die einzelnen Nadeln vorzugsweise nicht als einzelne Nadeln zu erkennen sind, so dass ein Andrücken des Mikronadelarrays gegen die Haut eines Patienten mit Spritzenphobie keine Angstzustände auslöst.

Vorzugsweise ist die Schutzhülle 1 aus einem undurchsichtigen, selbsttragenden und/oder flexiblen Material hergestellt, so dass die Nadel 10 unter der Schutzhülle 1 verdeckt bleibt und nicht sichtbar ist. Eine derartige Schutzhülle 1 hat zudem den Vorteil, dass sie für die Punktion nicht abgenommen werden muss und die Nadel 10 direkt die Schutzhülle 1 durchstechen kann, wodurch das distale Ende der Schutzhülle mit den Mikronadeln 2 entlang der Nadel 10 in Richtung der Spritze verschoben wird. Wenn die Punktion beendet wird, wird die Nadel 10 aus der Haut zurückgezogen, wodurch das distale Ende der flexiblen Schutzhülle 1 entlang der Nadel in die distale Richtung verschoben wird (weg von der Spritze) und die Nadel 10 vorzugsweise wieder vollständig umhüllt. Somit wird gewährleistet, dass die Nadel 10 wieder geschützt in der Nadelabdeckung liegt. Zwecknäßigerweise dient die Umhüllung dann auch gleichzeitig als Schutz gegen Stichverletzung bei der Entsorgung.

Als bevorzugte Materialien für die Schutzhülle 1 werden Kunststoffe oder Gummis mit einer entsprechenden Elastizität und Festigkeit verwendet. Vorzugsweise wird die Festigkeit der Schutzhülle so gewählt, dass sie groß genug ist, die Nadel sicher zu umhüllen und eine Akupunktur bzw. Medikamentenabgabe zu gewährleisten, jedoch zumindest am distalen Ende der Schutzhülle klein genug ist, damit sie von der Punktionsnadelspitze 11 sicher und ohne zu großen Kraftaufwand durchstochen werden kann.

Damit die Nadel das distale Ende der Umhüllung 1 durchstechen kann, ist die Umhüllung beispielsweise mit einer Einrichtung versehen, die beim Ausüben einer in Axialrichtung der Nadel wirkenden Druckkraft beim Erreichen einer Grenzkraft die Umhüllung 1 von der Nadelspitze 11 entfernt, so dass die Nadelspitze 11 freigelegt wird. Dies kann beispielsweise durch die Anbringung der Umhüllung 1 am Spritzenkörper 20 erfolgen. Hierzu kann z.B. eine Rutschkupplung vorgesehen sein, die ermöglicht, dass das proximale Ende der Umhüllung bei Erreichen einer vorbestimmten Kraft (Grenzkraft) entlang des Spritzenkörpers 20 gleitet. Alternativ dazu kann eine Feder vorgesehen sein, gegen die die Umhüllung 1 während der Akupunktur bzw. Medikamentenabgabe mit Druck einwirkt, diese jedoch nicht wesentlich komprimiert, so dass die Nadel 10 abgedeckt bleibt. Erst wenn die Grenzkraft erreicht und übertroffen wird, durchsticht die Nadel 10 das distale Ende der Umhüllung 1 und wird freigelegt. Das Material der Umhüllung 1 selbst kann auch derart steif ausgewählt und/oder ausgebildet werden, dass es während der Akupunktur bzw. Medikamentenabgabe die Form der Umhüllung 1 im Wesentlichcn auftechterhält, während es beim Übersteigen der Grenzkraft nachgibt, z.B. einknickt, und so die Nadelspitze 11 freigelegt wird.

Wie oben beschrieben, kann die erfindungsgemäße Nadelabdeckung 42 mit der Schutzhülle auf handelsübliche Spritzen angebracht werden.

Erfindungsgemäß kann die Nadelabdeckung 42 auch bereits vorinstalliert als Punktionssystem bzw. Set realisiert sein. Dies ist besonders vorteilhaft, wenn das zu injizierende Medikament bereits in einer Spritze zum fertigen Gebrauch schon aufgezogen ist, wie es beispielsweise bei Thrombosespritzen der Fall ist.

Die Verwendung der erfindungsgemäßen Nadelabdeckung 42 bzw. des erfindungsgemäßen Punktionssets kann wie folgt beschrieben werden. Der Anwender, z.B. Arzt oder der Patient selbst, nimmt die Spritze mit der erfindungsgemäßen Nadelabdeckung in die Hand und drückt die Vorderseite der Schutzhülle 1 mit dem Mikronadelarray an der Punktionsstelle leicht gegen die Haut. Dadurch dringen die Mikronadeln 2 leicht in die oberste Hautschicht ein, wodurch ein Akupunktureffekt und/oder eine Medikamentenabgabe erzielt wird und die Schmerzempfindlichkeit lokal an der Punktionsstelle reduziert werden kann. Abhängig von der Person und der Körperstelle wird eine gewisse Zeit gewartet, so dass sich der Betäubungseffekt einstellen kann. Danach wird höherer Druck gegen die Spritze ausgeübt, so dass die Nadelspitze 11 der Punktionsnadel 10 die Schutzhülle 1 am distalen Ende, d.h. am Ende, an dem das Mikronadelarray angebracht ist, durchsticht und schließlich in die Haut eindringen kann. Die Punktion selbst erfolgt nun unter vermindertem Schmerzempfinden aufgrund des eingestellten schmerzlindernden Akupunktureffekts und/oder des verabreichten Medikaments.

Zusätzlich zum Akupunktureffekt kann das Mikronadelarray auch dazu verwendet werden, um ein Lokalanästhetikum mittels der Mikronadeln unter die Hautoberfläche einzubringen, so dass die Wirkung des Lokalanästhetikums erhöht bzw. beschleunigt wird. Das Lokalanästhetikum kann entweder kurz vor der Injektion auf die Haut oder auf das Mikronadelarray aufgebracht werden oder kann schon auf dem Mikronadelarray aufgebracht und von einer Abdeckung abgedeckt sein, wobei die Abdeckung kurz vor der Punktion abgenommen wird. Es kann auch vorteilhaft sein, dass zumindest einige der Mikronadeln hohl bzw. porös ausgebildet und mit einem geeigneten Lokalanästhetikum gefüllt oder vermischt sind, so dass das Lokalanästhetikum unter die Hautschicht eingebracht wird, sobald die Mikronadeln in die Hautschicht eindringen.

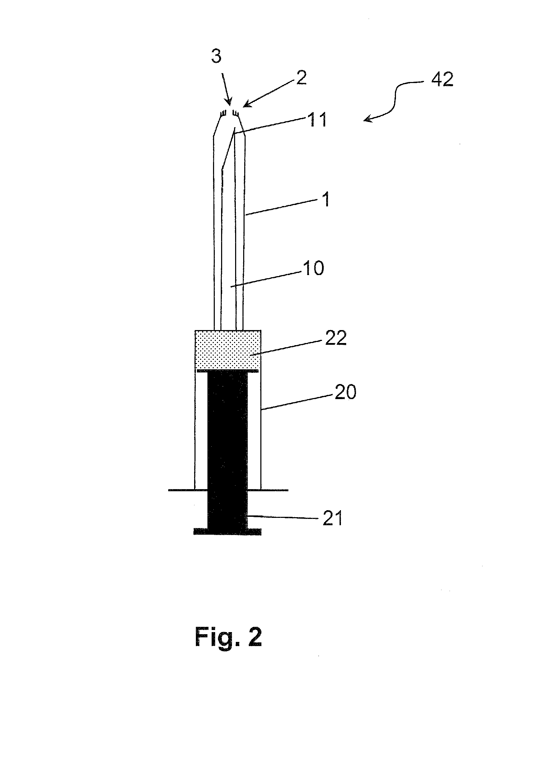

Fig. 2 zeigt eine Ausführungsform ähnlich der in Fig. 1 gezeigten, jedoch mit dem Unterschied, dass die erfindungsgemäße Schutzhülle 1 an der Stelle der Punktionsnadelspitze 11 eine kleine Öffnung 3 aufweist. Abhängig von der Größe der Punktionsnadel bzw. des Materials der Schutzhülle kann es vorteilhaft sein, eine Öffnung 3 bereitzustellen. So ist es bekannt, dass durch Einstechen in Gewebe oder Material die Schärfe der Nadelspitze zum Teil verloren gehen kann, wodurch die Punktion wiederum schmerzhafter werden kann. Aus diesem Grund werden Punktionsnadeln nach dem Entnehmen von Medikamenten aus so genannten Durchstichflaschen (zu durchstechender Gummiverschluss) vor der eigentlichen Injektion gewechselt. In der Ausführungsform mit der Öffnung 3 kann ein abstumpfen der Nadelspitze verhindert werden. In dieser Ausführungsformn ist die Öffnung 3 vorzugsweise so angeordnet, dass die Punktionsnadel 10 die Schutzhülle selbst nicht durchstechen muss, sondern bei der Punktion durch die Öffnung ausfährt.

Die Erfindung umfasst ebenfalls die genauen oder exakten Ausdrücke, Merkmale, numerischen Werte oder Bereiche usw., wenn vorstehend oder nachfolgend diese Ausdrücke, Merkmale, numerischen Werte oder Bereiche im Zusammenhang mit Ausdrücken wie z.B. "etwa, ca., um, im Wesentlichen, im Allgemeinen, zumindest, mindestens" usw. genannt wurden (also "etwa 3" soll ebenfalls "3" oder "im Wesentlichen radial" soll auch "radial" umfassen).

## Patentansprüche

1. Nadelabdeckung (42) für Punktionsnadeln (10) mit:
einer Schutzhülle (1) zum Umhüllen zumindest einer Nadelspitze (11) der Punktionsnadel (10), wobei die Schutzhülle eine zur Nadelspitze gerichtete Innenseite und eine von der Nadelspitze (11) weg gerichtete Außenseite aufweist, **dadurch gekennzeichnet, dass**
die Schutzhülle (1) an ihrer Außenseite mindestens ein Array von Mikronadeln (2) aufweist.

2. Nadelabdeckung nach Anspruch 1, wobei die Schutzhülle (1) ein ausreichend steifes Material bzw. eine ausreichend steife Konstruktion aufweist, dass damit die Mikronadeln (2) des Arrays zur Akupunktur und/oder Medikamentenabgabe in die Haut eindringen können, ohne dass die Nadelspitze (11) die Schutzhülle durchsticht.

3. Nadelabdeckung nach Anspruch 1 oder 2, wobei die Schutzhülle (1) eine Einrichtung aufweist, die beim Ausüben einer auf die Nadelspitze (11) in Axialrichtung wirkenden Druckkraft beim Überschreiten einer Grenzkraft die Nadelspitze (11) freilegt.

4. Nadelabdeckung einem der vorhergehenden Ansprüche, wobei die Mikronadeln (2) massiv, porös oder hohl sind.

5. Nabelabdeckung nach einem der vorhergehenden Ansprüche, wobei die Schutzhülle (1) dimensioniert ist, um handelsübliche Punktionsnadeln (10) zu umhüllen.

6. Nadelabdeckung nach einem der vorhergehenden Ansprüche, wobei die Länge der Mikronadeln (2) so bemessen ist, dass sie nicht in die Dermis der Haut mit den Schmerzrezeptoren und Gefäße eindringen, wenn die Mikronadeln (2) gegen die Haut gedrückt werden.

7. Nadelabdeckung nach einem der vorhergehenden Ansprüche, wobei die Schutzhülle (1) die Punktionsnadel (10) vollständig umhüllt, vorzugsweise vollständig fluiddicht umhüllt und von der Nadelspitze (11) durchstochen werden kann, wenn die Nadelspitze gegen ein Objekt gedrückt wird.

8. Nadelabdeckung nach einem der vorhergehenden Ansprüche, wobei die Schutzhülle (1) eine Öffnung (3) aufweist, durch die die Nadel sich erstreckt, wenn die Nadelspitze (11) gegen ein Objekt gedrückt wird.

9. Nadelabdeckung nach einem der vorhergehenden Ansprüche, wobei auf der Außenseite der Schutzhülle (1), vorzugsweise im Bereich des Mikronadelarrays ein Medikament vorhanden ist.

10. Nadelabdeckung nach einem der vorhergehenden Ansprüche, wobei die Mikronadeln (2) im Wesentlichen nur auf einer Fläche auf der Außenseite angebracht sind, an der sich die Nadelspitze (11) durch die Schutzhülle (1) erstreckt, wenn die Nadelspitze gegen ein Objekt gedrückt wird.

11. Nadelabdeckung nach einem der vorhergehenden Ansprüche, wobei die Mikronadeln (2) eine Länge im Bereich von 10 µm bis 900 µm, einen Durchmesser im Bereich von 4 µm bis 160 µm und einen Spitzendurchmesser im Bereich von 0,2 µm bis 30 µm haben.

12. Punktionssystem mit:
einer Spritze, aufweisend einen Spritzenkörper (20) mit einem Hohlraum und einem darin beweglichen Kolben (21);
einer Punktionsnadel (10) die an dem Spritzenlcörper angebracht bzw. anbringbar ist; und
einer Nadelabdeckung (1) nach einem der vorhergehenden Ansprüche, die an der Spritze und/oder Punktionsnadel (10) angebracht bzw. anbringbar ist, so dass die Schutzhülle (1) die Punktionsnadel abdeckt.

13. Verfahren zur Durchführung einer schmerzverminderten Haut-Punktion mit den Schritten:
Bereitstellen eines Punktionssystems nach Anspruch 12;
leichtes Andrücken der Schutzhülle (1) gegen die Haut, so dass die Mikronadeln (2) in die Haut eindringen;
anschließend stärkeres Andrücken der Schutzhülle (1) gegen die Haut, so dass sich die Nadelspitze (11) durch die Schutzhülle (1) erstreckt und in Haut eindringen kann.
